# EUROPÄISCHE PATENTSCHRIFT

(11) **EP 2 240 456 B1**
(45) Veröffentlichungstag und Bekanntmachung des Hinweises auf die Patenterteilung: **10.08.2011**
(21) Anmeldenummer: 09705420.9
(22) Anmeldetag: 15.01.2009
(51) Int. Cl.: C07D 251/10

(54) **VERFAHREN ZUR HERSTELLUNG VON 3, 6-DIHYDRO-L, 3, 5-TRIAZINDERIVATEN AUS METFORMIN- UND PARALDEHYDDERIVATEN**
METHOD FOR THE PRODUCTION OF 3, 6-DIHYDRO-L, 3, 5-TRIAZINE DERIVATIVES FROM METFORMIN AND PARALDEHYDE DERIVATIVES
PROCÉDÉ DE PRODUCTION DE DÉRIVÉS DE 3, 6-DIHYDRO-L, 3, 5-TRIAZINE À PARTIR DE DÉRIVÉS DE METFORMINE ET PARALDÉHYDES

(30) Priorität: 02.02.2008 DE 102008007314
(43) Veröffentlichungstag der Anmeldung: 20.10.2010
(73) Patentinhaber: POXEL SAS, 69007 Lyon (FR)
(72) Erfinder: HELMREICH, Matthias, 69120 HEIDELBERG (DE); BRANDNER, Mike, 64521 GROSS-GERAU (DE)
(74) Vertreter: Tezier Herman, Béatrice
(86) Internationale Anmeldenummer: PCT/EP2009/000212
(87) Internationale Veröffentlichungsnummer: WO 2009/095159

(56) Entgegenhaltungen:
- EP-A- 1 574 503
- WO-A-01/55122

## Beschreibung

Die Erfindung betrifft ein Verfahren zur Herstellung von Verbindungen der Formel I 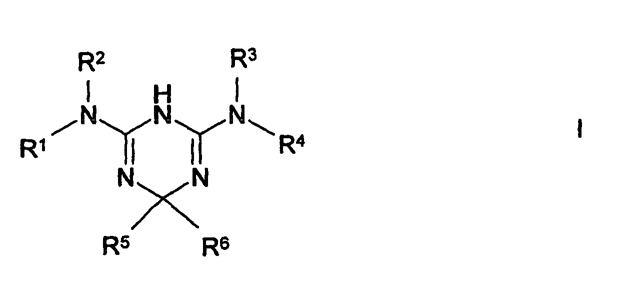 worin
- R¹, R²: jeweils unabhängig voneinander H oder A,
- R³, R⁴: jeweils unabhängig voneinander H, A, Alkenyl mit 2-6 C- Atomen, Alkinyl mit 2-6 C-Atomen, Ar oder Het,
- R⁵ und R⁶: zusammen auch Alkylen mit 2, 3, 4 oder 5 C-Atomen,
- R⁵, R⁶: jeweils unabhängig voneinander H, A, (CH₂)ₙAr, (CH₂)ₘOAr, (CH₂)ₘOA oder (CH₂)ₘOH,
- R⁵ und R⁶: zusammen auch Alkylen mit 2, 3, 4 oder 5 C-Atomen, worin eine CH₂-Gruppe durch O, NH oder NA ersetzt sein kann und/oder worin 1 H-Atom durch OH ersetzt sein kann,
- Ar: unsubstituiertes oder ein-, zwei- oder dreifach durch Hal, A, OA, OH, COOH, COOA, CN, NH₂, NHA, NA₂, SO₂A und/oder COA substituiertes Phenyl, Naphthyl oder Biphenyl,
- Het: einen ein-, zwei- oder dreikernigen gesättigten, ungesättigten oder aromatischen Heterocyclus mit 1 bis 4 N-, 0- und/oder S-Atomen, der unsubstituiert oder ein-, zwei- oder dreifach durch Hal, A, OH, OA, NH₂, (CH₂)ₙAr, NHA, NA₂, COOH, COOA und/oder =O (Carbonylsauerstoff) substituiert sein kann,
- A: unverzweigtes oder verzweigtes Alkyl mit 1-10 C-Atomen, worin 1-7 H-Atome durch F ersetzt sein können oder cyclisches Alkyl mit 3-7 C-Atomen,
- Hal: F, Cl, Br oder I,
- m: 1, 2, 3, 4, 5 oder 6,
- n: 0, 1 oder 2,
bedeuten,
sowie ihrer Säureadditionssalze,
das die Umsetzung einer Verbindung der Formel II 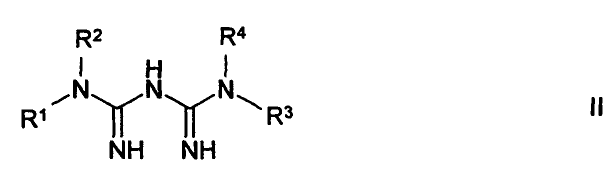 worin
- R¹, R², R³, R⁴: die oben angegebenen Bedeutungen haben,
mit einer Verbindung der Formel III 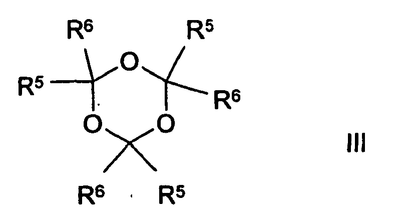 worin
- R⁵, R⁶: die oben angegebenen Bedeutungen haben,
umfasst.

Bekannt sind andere Verfahren zur Herstellung von Verbindungen der Formel I aus der EP 1 250 328 B1.

Die Verbindungen der Formel I sind nützlich bei der Behandlung von mit dem Insulinresistenzsyndrom verbundenen Erkrankungen.

Überraschenderweise ergaben Untersuchungen im Rahmen der Synthese von Dihydro-1,3,5-triazinaminderivaten, daß die Verbindungen der Formel I im Vergleich zum Stand der Technik in wenigstens vergleichbarer oder höherer Ausbeute erhalten werden können, wobei als entscheidende Vorteile dabei erheblich kürzere Reaktionszeit und weniger Abfallprodukte zu nennen sind. Das bedeutet in der Konsequenz auch einen erheblich geringeren Energieverbrauch.

So wird im erfindungsgemäßen Verfahren pro Molekül an gebildeter Verbindung der Formel I ein Molekül Wasser freigesetzt.
Im Verfahren des Standes der Technik werden pro Molekül an gebildeter Verbindung der Formel I zwei Moleküle Alkohol freigesetzt.

Nach dem erfindungsgemäßen Verfahren wird insbesondere die Verbindung 4-Amino-3,6-dihydro-2-dimethylamino-6-methyl-1,3,5-triazin hergestellt.

Vor- und nachstehend haben die Reste R¹, R², R³, R⁴, R⁵, R⁶ die bei der Formel I angegebenen Bedeutungen, sofern nicht ausdrücklich etwas anderes angegeben ist.

Formel I umfasst auch die optisch aktiven Formen (Stereoisomeren), wie die Enantiomeren.

Metformin als bevorzugtes Edukt hat die Struktur

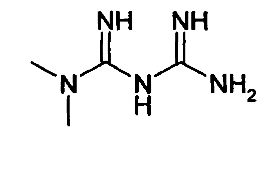

A bedeutet Alkyl, dieses ist unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.
A bedeutet weiterhin bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl.
A bedeutet ganz besonders bevorzugt Methyl.
Cyclisches Alkyl (Cycloalkyl) bedeutet vorzugsweise Cyclopropyl, Cyclobutyl, Cylopentyl, Cyclohexyl oder Cycloheptyl.

Alkenyl hat 2, 3, 4, 5 oder 6 C-Atome und bedeutet vorzugsweise Vinyl oder Propenyl.
Alkinyl hat 2, 3, 4, 5 oder 6 C-Atome und bedeutet vorzugsweise C=CH oder C=C-CH₃.

Ar bedeutet z.B. o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Hydroxyphenyl, o-, m- oder p-Aminophenyl, o-, m- oder p-(N-Methylamino)-phenyl, o-, m- oder p-(N-Methylamino-carbonyl)-phenyl, o-, m- oder p-Methoxyphenyl, o-, m- oder p-Ethoxyphenyl, o-, m- oder p-Ethoxycarbonylphenyl, o-, m- oder p-(N,N-Dimethylamino)-phenyl, o-, m- oder p-(N-Ethylamino)-phenyl, o-, m- oder p-(N,N-Diethylamino)-phenyl, o-, m- oder p-Fluorphenyl, o-, m- oder p-Bromphenyl, o-, m- oder p- Chlorphenyl, o-, m- oder p-(Methylsulfonyl)-phenyl, o-, m- oder p-Cyanphenyl, o-, m- oder p-Carboxyphenyl, o-, m- oder p-Methoxycarbonylphenyl, o-, m- oder p-Acetylphenyl weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,4-oder 2,5-Dinitrophenyl, 2,5- oder 3,4-Dimethoxyphenyl, 3-Amino-4-chlor-, 2-Amino-3-chlor-, 2-Amino-4-chlor-, 2-Amino-5-chlor- oder 2-Amino-6-chlorphenyl, 2,3-Diaminophenyl, 2,3,4-, 2,3,5-, 2,3,6-, 2,4,6- oder 3,4,5-Trichlorphenyl, 2,4,6-Trimethoxyphenyl, 2-Hydroxy-3,5-dichlorphenyl, p-lodphenyl, 3,6-Dichlor-4-aminophenyl, 4-Fluor-3-chlorphenyl, 2-Fluor-4-bromphenyl, 2,5-Difluor-4-bromphenyl, 3-Brom-6-methoxyphenyl, 3-Chlor-6-methoxyphenyl, 3-Fluor-4-methoxyphenyl, 3-Amino-6-methylphenyl oder 2,5-Dimethyl-4-chlorphenyl.
Ar bedeutet besonders bevorzugt Phenyl, Hydroxyphenyl oder Methoxyphenyl.

Het bedeutet, ungeachtet weiterer Substitutionen, z.B. 2- oder 3-Furyl, 2-oder 3-Thienyl, 1-, 2- oder 3-Pyrrolyl, 1-, 2, 4- oder-5-Imidazolyl, 1-, 3-, 4-oder 5-Pyrazolyl, 2-, 4- oder 5-Oxazolyl, 3-, 4- oder 5-lsoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4-oder-5-yl, 1,2,4-Triazol-1-, -3- oder 5-yl, 1- oder 5-tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, Indazolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyi, 3-, 4-, 5-, 6-, 7-oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzodioxol-5-yl, 1,4-Benzodioxan-6-yl, 2,1,3-Benzothiadiazol-4- oder -5-yl, 2,1,3-Benzoxadiazol-5-yl oder Dibenzofuranyl.
Die heterocyclischen Reste können auch teilweise oder vollständig hydriert sein.

Ungeachtet weiterer Substitutionen kann Het also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3-oder-4-pyrazolyl, 1,4-Dihydro-1-, -2-, -3-oder-4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-,-3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-,-2-,-3-, -4-, -5-, -6-, -7- oder-8-isochinolyl, 2-, 3-, 5-, 6-, 7-oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydro-benzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydrobenzofuranyl, 2,3-Dihydro-2-oxo-furanyl, 3,4-Dihydro-2-oxo-1H chinazolinyl, 2,3-Dihydro-benzoxazolyl, 2-Oxo-2,3-dihydro-benzoxazolyl, 2,3-Dihydro-benzimidazolyl, 1,3-Dihydroindol, 2-Oxo-1,3-dihydro-indol oder 2-Oxo-2,3-dihydro-benzimidazolyl.

Het bedeutet vorzugsweise unsubstituiertes oder ein-, zwei- oder dreifach durch A, COOA, Hal und/oder =O (Carbonylsauerstoff) substituiertes Piperidinyl, Piperazinyl, Pyrrolidinyl, Morpholinyl, Furyl, Thienyl, Pyrrolyl, Imidazolyl, Pyrazolyl, Oxazolyl, Isoxazolyl, Thiazolyl, Isothiazolyl, Pyridyl, Pyrimidinyl, Triazolyl, Tetrazolyl, Oxadiazolyl, Thiadiazolyl, Pyridazinyl, Pyrazinyl, Benzimidazolyl. Benzotriazolyl, Indolyl, Benzo[1,3]dioxolyl, Indazolyl oder Benzo[2,1,3]thiadiazolyl.
R¹, R² bedeuten vorzugsweise A.
R³, R⁴ bedeuten vorzugsweise H.
R⁵ bedeutet vorzugsweise H.
R⁶ bedeutet vorzugsweise A.

Ganz besonders bevorzugt bedeuten
- R¹, R²: Methyl,
- R³, R⁴: H
- R⁵: H,
- R⁶: Methyl.

Die Verbindungen mit der allgemeinen Formel (II) sind Biguanide, deren Synthese vom Durchschnittsfachmann beherrscht wird. Es werden beispielhaft einige Publikationen zitiert, in welchen die Synthese solcher Verbindungen beschrieben ist (FR1 537 604:FR 2 132 396; K.H. Slotta und R. Tschesche, Ber., 1929(62b), 1398; S.L. Shapiro, V.A. Parrino, E. Rogow und L. Freedman, J. Org. Chem., 1959(81), 3725; S.L. Shapiro, V.A. Parrino und L. Freedman, J. Org. Chem., 1959(81), 3728 und S.L. Shapiro, V.A. Parrino und L. Freedman, J. Org. Chem., 1959(81), 4636.

Die Umsetzung der Verbindungen II und III verläuft in einem geeigneten polaren Lösungsmittel, wie z.B. Alkohole wie Methanol, Ethanol, Isopropanol, n-Propanol, n-Butanol, iso-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Ester wie Ethylacetat oder Gemische der genannten Lösungsmittel.
Besonders bevorzugt ist Isobutanol, ferner Ethanol und Isopropanol.

Die Reaktionszeit liegt je nach den angewendeten Bedingungen zwischen einigen Minuten und 14 Tagen, besonders bevorzugt zwischen 3 und 12 Stunden; die Reaktionstemperatur zwischen etwa 50° und 150°, normalerweise zwischen 90° und 120°.

Die Umsetzung erfolgt in Gegenwart einer organischen oder anorganischen Säure. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, lsonicotinsäure, Methan- oder Ethansulfonsäure, Ethandisulfonsäure, 2-Hydroxyethansülfonsäure, Benzolsulfonsäure, p-Toluolsulfonsäure, Naphthalin-mono- und Disulfonsäuren, Laurylschwefelsäure. Weiterhin geeignet sind saure kationische lonentauscherharze, wie z.B. die kommerziell erhältlichen Dowex^{®} oder Amberlyst^{®} - Harze.
Ganz besonders bevorzugt ist p-Toluolsulfonsäure, ferner Salzsäure, Methansulfonsäure, Schwefelsäure oder Camphersulfonsäure oder saure kationische lonentauscherharze, z.B. Dowex^{®} 50, Amberlyst^{®} 15 oder Dowex^{®} DR-2030.
Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und Disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation.

### Herstellung von 4-Amino-3,6-dihydro-2-dimethylamino-6-methyl-1,3,5-triazin Hydrochlorid

### Vergleichsbeispiel

Eine Mischung von 250,2 g Metformin Hydrochlorid, 213,6 g Acetaldehyddiethylacetal und 12,5 g Toluol-4-sulfonsäure Monohydrat in 500 ml Isobutanol wird 40 Stunden unter Rückfluß erhitzt. Ein Teil des Lösungsmittels wird abdestilliert. Man kühlt auf 10° ab und trennt den weißen Niederschlag ab.

Man erhält 224,7 g (77,4 %) 4-Amino-3,6-dihydro-2-dimethylamino-6-methyl-1,3,5-triazin Hydrochlorid.

### Beispiel 1

Eine Mischung von 1002,6 g Metformin Hydrochlorid, 359,1 g Paraldehyd und 51,6 g Toluol-4-sulfonsäure Monohydrat in 2405,9 g Isobutanol wird 6 Stunden unter Rückfluß erhitzt. Ein Teil des Lösungsmittels wird abdestilliert. Man kühlt auf 12° ab und trennt den weißen Niederschlag ab. Man erhält 953,8 g (81,4 %) 4-Amino-3,6-dihydro-2-dimethylamino-6-methyl-1,3,5-triazin Hydrochlorid.

### Beispiel 2

Eine Mischung von 100,1 g Metformin Hydrochlorid, 36,5 g Paraldehyd und 4 g Dowex DR-2030 in 237,8 Isobutanol wird 6 Stunden unter Rückfluß erhitzt. Anschließend wird der Katalysator abfiltriert und ein Teil des Lösungsmittels abdestilliert. Den rest der Lösung kühlt man auf 10-15 °C ab und trennt den weißen Niederschlag ab. Man erhält 93,5 g (80,7%) 4-Amino-3,6-dihydro-2-dimethylamino-6-methyl-1,3,5-triazin hydrochlorid.

## Patentansprüche

1. Verfahren zur Herstellung von Verbindungen der Formel I 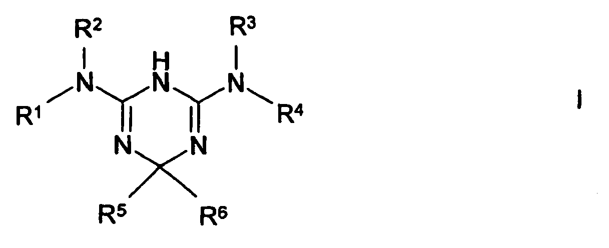 worin
R¹, R² jeweils unabhängig voneinander H oder A,
R³, R⁴ jeweils unabhängig voneinander H, A, Alkenyl mit 2-6 C- Atomen, Alkinyl mit 2-6 C-Atomen, Ar oder Het,
R⁵ und R⁶ zusammen auch Alkylen mit 2, 3, 4 oder 5 C-Atomen,
R⁵,R⁶ jeweils unabhängig voneinander H, A, (CH₂)ₙAr, (CH₂)ₘOAr, (CH₂)ₘOA oder (CH₂)ₘOH,
R⁵ und R⁶ zusammen auch Alkylen mit 2, 3, 4 oder 5 C-Atomen, worin eine CH₂-Gruppe durch O, NH oder NA ersetzt sein kann und/oder worin 1 H-Atom durch OH ersetzt sein kann,
Ar unsubstituiertes oder ein-, zwei- oder dreifach durch Hal, A, OA, OH, COOH, COOA, CN, NH₂, NHA, NA₂, SO₂A und/oder COA substituiertes Phenyl, Naphthyl oder Biphenyl,
Het einen ein-, zwei- oder dreikernigen gesättigten, ungesättigten oder aromatischen Heterocyclus mit 1 bis 4 N-, O- und/oder S-Atomen, der unsubstituiert oder ein-, zwei- oder dreifach durch Hal, A, OH, OA, NH₂, (CH₂)ₙAr, NHA, NA₂, COOH, COOA und/oder =O (Carbonylsauerstoff) substituiert sein kann,
A unverzweigtes oder verzweigtes Alkyl mit 1-10 C- Atomen, worin 1-7 H-Atome durch F ersetzt sein können oder cyclisches Alkyl mit 3-7 C-Atomen,
Hal F, Cl, Br oder I,
m 1, 2, 3, 4, 5 oder 6,
n 0, 1 oder 2,
bedeuten,
sowie ihrer Säureadditionssalze,
das die Umsetzung einer Verbindung der Formel II 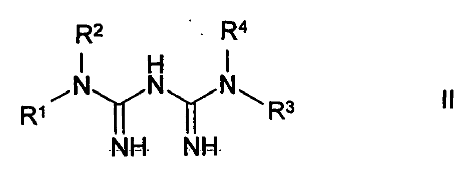 worin
R¹, R², R³, R⁴ die oben angegebenen Bedeutungen haben,
mit einer Verbindung der Formel III 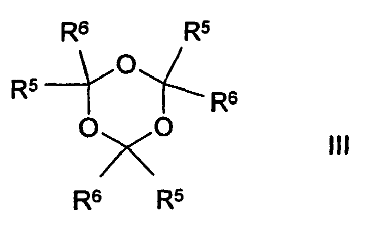 worin
R⁵, R⁶ die oben angegebenen Bedeutungen haben,
umfasst.

2. Verfahren nach Anspruch 1, wobei die Umsetzung in Gegenwart einer organischen oder anorganischen Säure oder eines sauren kationischen lonentauscherharzes erfolgt.

3. Verfahren nach Anspruch 1 oder 2, wobei die Umsetzung in Gegenwart von para-Toluolsulfonsäure oder eines sauren kationischen lonentauscherharzes erfolgt.

4. Verfahren nach einem oder mehreren der Ansprüche 1-3, wobei die Umsetzung in einem polaren Lösungsmittel erfolgt.

5. Verfahren nach einem oder mehreren der Ansprüche 1-4, wobei die Umsetzung in Isobutanol erfolgt.

6. Verfahren nach einem oder mehreren der Ansprüche 1-5, zur Herstellung von Verbindungen der Formel I, worin
R¹, R² A
bedeuten.

7. Verfahren nach einem oder mehreren der Ansprüche 1-6, zur Herstellung von Verbindungen der Formel I,
worin
R³, R⁴ H
bedeuten.

8. Verfahren nach einem oder mehreren der Ansprüche 1-7, zur Herstellung von Verbindungen der Formel I,
worin
R⁵ H,
R⁶ A
bedeuten.

9. Verfahren nach einem oder mehreren der Ansprüche 1-8, zur Herstellung von Verbindungen der Formel I,
worin
R¹, R² Methyl,
R³, R⁴ H,
R⁵ H,
R⁶ Methyl
bedeuten.

## Claims

1. Process for the preparation of compounds of the formula I 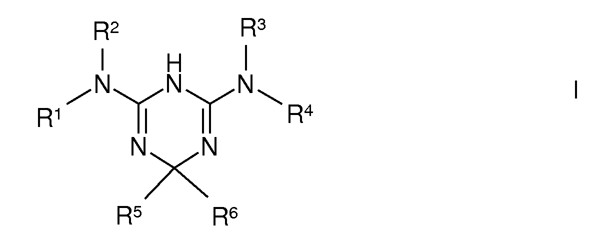 in which
R¹, R² each, independently of one another, denote H or A,
R³, R⁴ each, independently of one another, denote H, A, alkenyl having 2-6 C atoms, alkynyl having 2-6 C atoms, Ar or Het,
R⁵ and R⁶ together also denote alkylene having 2, 3, 4 or 5 C atoms,
R⁵, R⁶ each, independently of one another, denote H, A, (CH₂)ₙAr, (CH₂)ₘOAr, (CH₂)ₘOA or (CH₂)ₘOH,
R⁵ and R⁶ together also denote alkylene having 2, 3, 4 or 5 C atoms, in which one CH₂ group may be replaced by O, NH or NA and/or in which 1 H atom may be replaced by OH,
Ar denotes phenyl, naphthyl or biphenyl, each of which is un- substituted or mono-, di- or trisubstituted by Hal, A, OA, OH, COOH, COOA, CN, NH₂, NHA, NA₂, SO₂A and/or COA,
Het denotes a mono-, bi- or tricyclic saturated, unsaturated or aromatic heterocycle having 1 to 4 N, O and/or S atoms, which may be unsubstituted or mono-, di- or trisubstituted by Hal, A, OH, OA, NH₂, (CH₂)ₙAr, NHA, NA₂, COOH, COOA and/or =O (carbonyl oxygen),
A denotes unbranched or branched alkyl having 1-10 C atoms, in which 1-7 H atoms may be replaced by F, or cyclic alkyl having 3-7 C atoms,
Hal denotes F, Cl, Br or I,
m denotes 1, 2, 3, 4, 5 or 6,
n denotes 0, 1 or 2,
and acid-addition salts thereof,
which comprises the reaction of a compound of the formula II 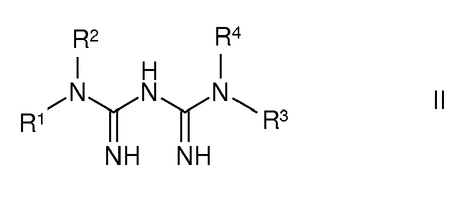 in which
R¹, R², R³, R⁴ have the meanings indicated above,
with a compound of the formula III 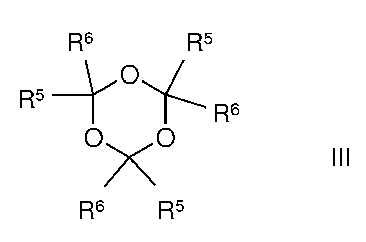 in which
R⁵, R⁶ have the meanings indicated above.

2. Process according to Claim 1, where the reaction is carried out in the presence of an organic or inorganic acid or an acidic cationic ion exchanger resin.

3. Process according to Claim 1 or 2, where the reaction is carried out in the presence of para-toluenesulfonic acid or an acidic cationic ion exchanger resin.

4. Process according to one or more of Claims 1-3, where the reaction is carried out in a polar solvent.

5. Process according to one or more of Claims 1-4, where the reaction is carried out in isobutanol.

6. Process according to one or more of Claims 1-5 for the preparation of compounds of the formula I
in which
R¹, R² denote A.

7. Process according to one or more of Claims 1-6 for the preparation of compounds of the formula I in which
R³, R⁴ denote H.

8. Process according to one or more of Claims 1-7 for the preparation of compounds of the formula I in which
R⁵ denotes H,
R⁶ denotes A.

9. Process according to one or more of Claims 1-8 for the preparation of compounds of the formula I in which
R¹, R² denote methyl,
R³, R⁴ denote H,
R⁵ denotes H,
R⁶ denotes methyl.

## Revendications

1. Procédé de préparation de composés de formule I 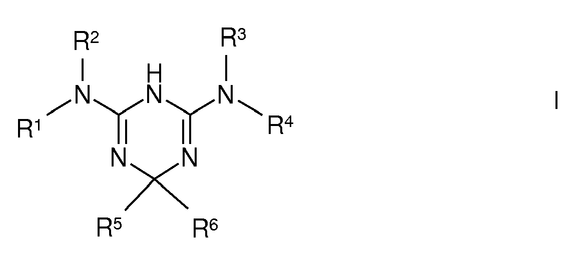 dans laquelle
R¹, R² représentent chacun indépendamment H ou A,
R³, R⁴ représentent chacun indépendamment H, A, un groupe alcényle ayant 2-6 atomes de C, un groupe alcynyle ayant 2-6 atomes de C, Ar ou Het,
R⁵ et R⁶ représentent aussi ensemble un groupe alkylène ayant 2, 3, 4 ou 5 atomes de C,
R⁵, R⁶ représentent chacun indépendamment H, A, (CH₂)ₙAr, (CH₂)ₘOAr, (CH₂)ₘOA ou (CH₂)ₘOH,
R⁵ et R⁶ représentent aussi ensemble un groupe alkylène ayant 2, 3, 4 ou 5 atomes de C, dans lequel un groupe CH₂ peut être remplacé par O, NH ou NA et/ou dans lequel un atome de H peut être remplacé par OH,
Ar représente un groupe phényle, naphtyle ou biphényle, non substitué ou mono-, di- ou trisubstitué par Hal, A, OA, OH, COOH, COOA, CN, NH₂, NHA, NA₂, SO₂A et/ou COA,
Het représente un hétérocycle saturé, insaturé ou aromatique, mono-, bi- ou tricyclique, ayant 1 à 4 atomes de N, O et/ou S, qui peut être non substitué ou mono-, di- ou trisubstitué par Hal, A, OH, OA, NH₂, (CH₂)ₙAr, NHA, NA₂, COOH, COOA et/ou =O (oxygène de carbonyle),
A représente un groupe alkyle non ramifié ou ramifié ayant 1 - 10 atomes de C, dans lequel 1-7 atomes de H peuvent être remplacés par F, ou un groupe alkyle cyclique ayant 3-7 atomes de C,
Hal représente F, CI, Br ou I,
m représente 1, 2, 3, 4, 5 ou 6,
n représente 0, 1 ou 2,
et leurs sels d'addition acide,
comprenant la réaction d'un composé de formule II 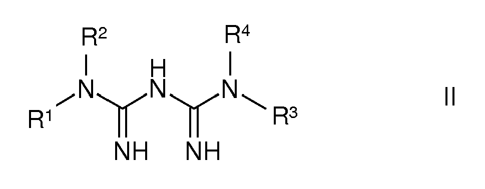 dans laquelle
R¹, R², R³, R⁴ ont les définitions indiquées ci-dessus,
avec un composé de formule III 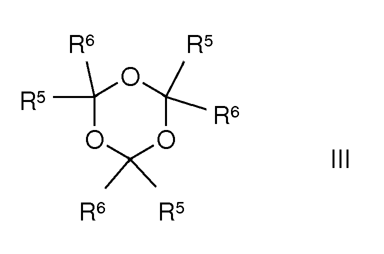 dans laquelle
R⁵, R⁶ ont les définitions indiquées ci-dessus.

2. Procédé selon la revendication 1, dans lequel la réaction est réalisée en présence d'un acide organique ou inorganique ou d'une résine échangeuse d'ions cationique acide.

3. Procédé selon la revendication 1 ou 2, dans lequel la réaction est réalisée en présence d'acide para-toluènesulfonique ou d'une résine échangeuse d'ions cationique acide.

4. Procédé selon l'une quelconque des revendications 1-3, dans lequel la réaction est réalisée dans un solvant polaire.

5. Procédé selon l'une quelconque des revendications 1-4, dans lequel la réaction est réalisée dans l'isobutanol.

6. Procédé selon l'une quelconque des revendications 1-5 pour la préparation de composés de formule I
dans laquelle
R¹, R² représentent A.

7. Procédé selon l'une quelconque des revendications 1-6 pour la préparation de composés de formule I dans laquelle
R³, R⁴ représentent H.

8. Procédé selon l'une quelconque des revendications 1-7 pour la préparation de composés de formule I dans laquelle
R⁵ représente H,
R⁶ représente A.

9. Procédé selon l'une quelconque des revendications 1-8 pour la préparation de composés de formule I dans laquelle
R¹, R² représentent un groupe méthyle,
R³, R⁴ représentent H,
R⁵ représente H,
R⁶ représente un groupe méthyle.
